(19) Europäisches Patentamt
European Patent Office
Office européen des brevets

(11) Veröffentlichungsnummer : **0 060 810**
**B1**

(12) **EUROPÄISCHE PATENTSCHRIFT**

(45) Veröffentlichungstag der Patentschrift : **17.10.84**

(51) Int. Cl.[3] : **A 61 K 31/66** // (A61K31/66, 31/415)

(21) Anmeldenummer : **82810112.1**

(22) Anmeldetag : **12.03.82**

(54) **Anthelmintika.**

(30) Priorität : **18.03.81 CH 1841/81**

(43) Veröffentlichungstag der Anmeldung : **22.09.82 Patentblatt 82/38**

(45) Bekanntmachung des Hinweises auf die Patenterteilung : **17.10.84 Patentblatt 84/42**

(84) Benannte Vertragsstaaten : **BE CH DE FR IT LI NL SE**

(56) Entgegenhaltungen :
**CH-A- 580 080**
**CH-A- 583 710**
**CH-A- 601 329**

(73) Patentinhaber : **CIBA-GEIGY AG**
**Postfach**
**CH-4002 Basel (CH)**

(72) Erfinder : **Boray, Joseph Coleman, Dr.**
**21 Bogota Avenue**
**Neutral Bay, N.S.W. 2089 (AU)**

**EP 0 060 810 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

**Beschreibung**

Die vorliegende Erfindung betrifft neue anthelmintische Mittel, die als aktive Komponente eine Wirkstoffkombination enthalten, und ihre Verwendung zur Bekämpfung von Helminthen, insbesondere von Nematoden, in Tieren.

Die den erfindungsgemässen Mitteln zugrundeliegenden Wirkstoffkombinationen weisen eine synergistische Wirksamkeit auf, welche die additive Wirkung der kombinierten Einzelwirkstoffe in vorteilhafter Weise übertrifft. Die erfindungsgemässen Wirkstoffkombinationen bestehen aus folgenden Einzelwirkstoffen :

Verbindung der Formel I

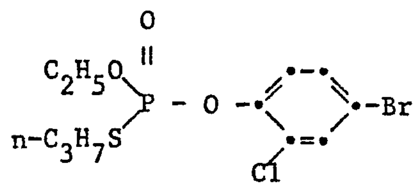

(I)

und Verbindung der Formel II

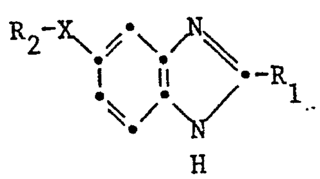

(II)

in welcher $R_1$ die Gruppe —NH—COO—$R_3$, worin $R_3$ einen Alkylrest mit 1 bis 4 Kohlenstoffatomen, vorzugsweise Methyl, darstellt, $R_2$ einen Alkylrest mit 1 bis 4 Kohlenstoffatomen, vorzugsweise n-Propyl oder gegebenenfalls durch Halogen oder Alkyl substituiertes Phenyl, vorzugsweise unsubstituiertes Phenyl, und X : O oder S oder —S(O)— bedeuten, einschliesslich der Säureadditionssalze der unter die Formel II fallenden Verbindungen.

Die als Wirkstoffkomponenten der erfindungsgemässen Mittel vorstehend beschriebenen Verbindungen sind individuell als anthelmintisch wirksam bekannt. Verbindungen der Formel I sind beispielsweise in der schweizerischen Patentschrift Nr. 601 329 und diejenigen der Formel II in Am. J. Vet. Res. *37*, 1285-86 und 1515-16 (1976), in Ann. Med. Vet. *120*, 515-19 (1976), in Vet. Rec. *99*, 267-70 (1976) und *101*, 260-63 (1977) und in der belgischen Patentschrift 793 358 beschrieben.

Die Kombination von verschiedenen chemischen Verbindungen zur Erzeugung synergistischer Wirkungsverstärkung für die Bekämpfung von Schädlingen erlangt durch die damit gegebene Möglichkeit, der immer häufiger in Erscheinung tretenden Resistenz der Parasiten gegenüber Einzelverbindungen wirksam entgegenzutreten, in zunehmendem Masse an Bedeutung. Eine wegen des Auftretens von Resistenzerscheinungen bei den Parasiten notwendig werdende Erhöhung der zum Einsatz gelangenden Wirkstoffmengen kann somit weitgehend vermieden werden. Die sich daraus ergebende Einsparung an Substanzmengen bringt sowohl wirtschaftliche als auch durch Verminderung der Umweltbelastung bedeutsame ökologische Vorteile mit sich. Für die Verabreichung von Anthelmintika an Nutztiere kann das eine Senkung der Applikationsdosis bedeuten, womit gleichzeitig eine Verminderung der Intoxikation der behandelten Tiere verbunden ist, so dass deren Nutzleistung während der Therapie nicht beeinträchtigt wird.

Der Wurmbefall von Haus- und Nutztieren ist weit verbreitet und führt häufig zur Hemmung des Wachstums und verminderter Nutzleistung der infestierten Tiere. Extrem starker Wurmbefall kann sogar den Tod der betroffenen Tiere zur Folge haben. Durch Wurmerkrankungen hervorgerufene Schäden nehmen vor allem bei epidemieartigem Auftreten des Befalls in Viehherden ein beträchtliches Ausmass an. Bekämpfung und Vorbeugung des Wurmbefalls von Nutztieren sind deshalb als vordringliche Aufgabe anzusehen, um derartige vor allem volkswirtschaftlich ins Gewicht fallende Schäden in der tierischen Produktion zu vermeiden. Ein besonderes Problem stellt dabei die sich in zunehmendem Masse entwickelnde Resistenz von tierparasitären Helminthen gegen gebräuchliche anthelmintische Einzelwirkstoffe dar. In dieser Hinsicht sind besonders die weitverbreiteten, vornehmlich in Schafen und Rindern parasitierenden Nematoden Haemonchus contortus und Trichostrongylus colubriformis zu nennen, die bereits weitgehend gegen Anthelmintika, wie die häufig verwendeten Benzimidazol-Derivate, resistent sind. Ausserdem sind bereits Kombinationen von Einzelwirkstoffen zur Bekämpfung von Helminthen in Tieren vorgeschlagen worden. Diese Kombinationen haben sich jedoch bisher als zuwenig

wirksam erwiesen, um den parasitären Wurmbefall von Tieren und die dadurch bedingten Schäden und Verluste in der Tierzucht in gewünschter Weise einzudämmen.

Es wurde nun gefunden, dass die in den erfindungsgemässen Mitteln enthaltenen Wirkstoffkombinationen, bestehend aus der Verbindung der Formel I und einer Verbindung der Formel II, eine hohe anthelmintische Wirksamkeit mit breitem Wirkungsspektrum gegen tierparasitäre Helminthen wie Nematoden, Cestoden und Trematoden entfalten. Dabei zeigt sich, dass die erfindungsgemässen Mittel vorzugsweise gegen Nematoden in Schafen und Rindern wirksam sind, was besonders ausgeprägt bei der Bekämpfung der Nematodenspecies Haemonchus contortus und Trichostrongylus colubriformis in Schafen der Fall ist.

Als wesentlicher Vorteil der erfindungsgemässen Mittel ist ihre Eigenschaft hervorzuheben, auch gegen resistente Helminthen gut wirksam zu sein.

Für die Anwendung der erfindungsgemässen Mittel ist besonders vorteilhaft, dass die Ihnen zugrundeliegenden Wirkstoffkombinationen bereits bei niederer Dosierung totale Wirkung gegen die bekämpften Helminthen erzielen. Toxische Nebenwirkungen, wie sie als Begleiterscheinungen von hohen therapeutischen Dosen auftreten können, werden auf diese Weise vermieden, so dass eine Beeinträchtigung der Entwicklung und der Nutzleistung der behandelten Tiere während der Therapie kaum in Erscheinung tritt.

Als bevorzugt in der Bekämpfung des Wurmbefalls von Tieren sind folgende Wirkstoffkombinationen anzusehen :

A) O-Aethyl-S-n-propyl-O-(2-chlor-4-bromphenyl)-phosphat mit entweder
B) [5-(n-Propylthio)-1H-benzimidazol-2-yl]-carbaminsäuremethylester (« Albendazol »)
oder
C) [5-(Phenylthio)-1H-benzimidazol-2-yl]-carbaminsäuremethylester (« Fenbendazol »)
oder
D) [5-n-Propoxy)-1H-benzimidazol-2-yl]-carbaminsäuremethylester (« Oxibendazol »)
oder
E) [5-Phenylsulfinyl-1H-benzimidazol-2-yl]-carbaminsäuremethylester (« Oxfendazol »).

Zur Entfaltung der synergistischen Wirkung der erfindungsgemässen Wirkstoffkombinationen ist für die Gewichtsverhältnisse von Verbindungen der Formel I zu Verbindungen der Formel II vorzugsweise der Bereich von 1 : 10 bis 10 : 1 massgebend. Ein Kombinationsverhältnis im Bereich von 1 : 5 bis 5 : 1 erweist sich vor allem beim Einsatz gegen resistente Helminthen als besonders vorteilhaft.

Die synergistische Wirksamkeit der den erfindungsgemässen Mitteln zugrundeliegenden Wirkstoffkombinationen wird anhand der in den nachfolgend beschriebenen Versuchen geprüften Wirkstoffkombinationen beispielhaft bewiesen.

Beispiel 1

Versuch an mit Benzimidazol-Resistenten Haemonchus contortus und Trichostrongylus colubriformis infizierten Schafen.

1.1 Testsubstanzen

Die Testsubstanzen wurden den Versuchstieren in Form von Drenchformulierungen verabreicht.

Albendazol : Handelsübliche flüssige Drenchformulierung enthaltend 19 mg/ml Wirkstoff (der Formel B, Seite 4).

Verbindung der Formel I : Drenchformulierung mit 25 %iger Wirkstoffkonzentration.

Zusammensetzung der verwendeten Drenchformulierung :

25,0 g Verbindung der Formel I
1,0 g Alkylarylpolyglykoläther (Emulgin® 286)
0,2 g tert.-Butylhydroxyanisol ad 104,6 g Di-n-butyladipat (Cetiol® B)
= 100 ml

1.2 Versuchsdurchführung

1.2.1 Infektion und Therapie

27 Schafe wurden artifiziell mit je 5 000 Larven ($L_3$) von Haemonchus contortus und mit je 12 000 Larven ($L_3$) von Trichostrongylus colubriformis infiziert.

Die Versuchstiere wurden in 5 Gruppen zu je 4 und 1 Gruppe zu 7 Individuen eingeteilt. 4 Wochen nach der Infizierung wurden die aus 4 Tieren bestehenden Gruppen mit den Testsubstanzen (Einzelwirkstoffe oder Gemische davon) intraruminal therapiert. Die 7 Schafe umfassende Gruppe blieb zur Kontrolle ohne Medikation.

1.2.2 Haltung der Versuchstiere

Die Versuchstiere wurden in mit Betonböden versehenen Gehegen gehalten und mit Futter bestehend aus 8 Teilen Weizen-Heu (wheaten hay), 1 Teil Leinsamenmehl und 1 % Mineral-Premix ad libitum gefüttert.

1.3 Auswertung

Vor sowie 7, 14, 28 und 42 Tage nach der Medikation wurden den Versuchstieren rektal Kotproben entnommen und darin die Anzahl der Nematodeneier mit einer standardisierten Flotationsmethode bestimmt.

(Siehe Tabellen Seite 5 ff.)

1.4 Tabelle

Resultate der Wirksamkeit der getesteten Substanzen und deren Gemische.

| Wirkstoffe | Dosis mg/kg | Anzahl Eier pro Gramm Kot bei einzelnen Versuchstieren | | | | | Reduktion der Eizahlen im Kot in % |
|---|---|---|---|---|---|---|---|
| | | vor Behandlung | nach Behandlung (Tage) | | | | |
| | | | 7 | 14 | 35 | 42 | |
| Albendazol | 2.3 | 8200 | 200 | 7800 | 8000 | 8200 | |
| | | 5000 | 400 | 5300 | 11700 | 9300 | |
| | | 10900 | 2600 | 13700 | 9800 | 10100 | |
| | | 4100 | 200 | 15200 | 3700 | 3100 | |
| | | Mittel 7050 | | | Mittel | 7675 | 0 |
| Albendazol | 4.6 | 4300 | 300 | 2700 | 1300 | 500 | |
| | | 4600 | 400 | 900 | 2400 | 1400 | |
| | | 8300 | 800 | 4500 | 4100 | 1500 | |
| | | 14400 | 2200 | 17700 | 30500 | 37600 | |
| | | Mittel 7900 | | | Mittel | 10250 | 0 |
| Verbindung Formel I | 10.0 | 3600 | 1400 | 4700 | 3600 | 4000 | |
| | | 2600 | 1200 | 2100 | 1400 | 5800 | |
| | | 5300 | 900 | 1200 | 1700 | 700 | |
| | | 1000 | 300 | 600 | 400 | 900 | |
| | | Mittel 3125 | | | Mittel | 2850 | 8.8 |

Tabelle (Fortsetzung)

| Wirkstoff | Dosis mg/kg | Anzahl Eier pro Gramm Kot bei einzelnen Versuchstieren | | | | | Reduktion der Eizahlen im Kot in % |
|---|---|---|---|---|---|---|---|
| | | vor Behandlung | nach Behandlung (Tage) | | | | |
| | | | 7 | 14 | 35 | 42 | |
| Albendazol/ | 2.3 | 2000 | 0 | 0 | 100 | 100 | |
| Verbindung | 10.0 | 1900 | 0 | 0 | 0 | 100 | |
| Formel I | | 700 | 0 | 0 | 0 | 0 | |
| | | 3200 | 0 | 0 | 200 | 300 | |
| | | Mittel 1950 | | | | Mittel 125 | 93.6 |
| Albendazol/ | 4.6 | 2200 | 0 | 0 | 0 | 0 | |
| Verbindung | 10.0 | 1900 | 0 | 0 | 0 | 0 | |
| Formel I | | 3500 | 0 | 0 | 0 | 0 | |
| | | 800 | 0 | 0 | 0 | 200 | |
| | | Mittel 2100 | | | | Mittel 50 | 97.6 |
| Kontrolle | - | 3700 | 7100 | 10900 | 15200 | 9800 | |
| | | 10400 | 11300 | 11100 | 5500 | 5300 | |
| | | 9300 | 12500 | 12800 | 10200 | 6600 | |
| | | 1800 | 4000 | 5300 | 3700 | 1800 | |
| | | 3800 | 4400 | 6800 | 4500 | 5000 | |
| | | 2400 | 4100 | 1900 | 4000 | 1900 | |
| | | 3500 | 5300 | 9800 | 18300 | 24300 | - |
| | | Mittel 4986 | | | | Mittel 7814 | |

Beispiel 2

Versuch an mit Benzimidazol-resistenten Haemonchus contortus und Trichostrongylus colubriformis infizierten Schafen.

2.1 Testsubstanzen

Die Testsubstanzen wurden den Versuchstieren in Form von Drenchformulierungen verabreicht.

Fenbendazol: Handelsübliche flüssige Drenchformulierung enthaltend 25 mg/ml Wirkstoff (der Formel C, Seite 4).

Verbindung der Formel I: Drenchformulierung mit 12,5 %iger Wirkstoffkonzentration.

Zusammensetzung der verwendeten Drenchformulierung:

12,5 g Verbindung der Formel I
1,0 g Sorbitantrioleat (Span® 85) ad
77,5 g Paraffinöl, dünnflüssig
= 100 ml

2.2 Versuchsdurchführung

2.2.1 Infektion und Therapie

30 Schafe wurden artifiziell mit je 5 000 Larven ($L_3$) von Haemonchus contortus und mit je 12 000 Larven ($L_3$) von Trichostrongylus colubriformis infiziert.
Die Versuchstiere wurden in 6 Gruppen zu je 4 und 1 Gruppe zu 6 Individuen eingeteilt. 4 Wochen nach der Infizierung wurden die aus 4 Tieren bestehenden Gruppen mit den Testsubstanzen (Einzelwirkstoffe oder Gemische davon) intraruminal therapiert. Die 6 Schafe umfassende Gruppe blieb zur Kontrolle ohne Medikation.

2.2.2 Haltung der Versuchstiere

Die Versuchstiere wurden in mit Betonböden versehenen Gehegen gehalten und mit Futter bestehend aus 8 Teilen Weizen-Heu (wheaten hay), 1 Teil Leinsamenmehl und 1 % Mineral-Premix ad libitum gefüttert.

2.3 Auswertung

14 bis 16 Tage nach der Medikation wurden die Versuchstiere geschlachtet, die adulten Nematoden aus den befallenen Magen- und Darmabschnitten ausgewaschen und gezählt.

(Siehe Tabellen Seite 8 ff.)

2.4 Tabelle

Resultate der Wirksamkeit der getesteten Substanzen und deren Gemische

| Wirkstoffe | Dosis mg/kg | Anzahl Nematoden bei einzelnen Versuchstieren 14 - 16 nach Behandlung | | Reduktion der Wurmzahl in % | |
|---|---|---|---|---|---|
| | | Haemonchus c. | Trichostrongylus c. | H.c. | T.c. |
| Fenbendazol | 2.5 | 100 | 2613 | 63,7 | 0 |
| | | 475 | 2088 | | |
| | | 650 | 1763 | | |
| | | 350 | 2800 | | |
| | | Mittel 395 | Mittel 2316 | | |
| Fenbendazol | 5.0 | 500 | 3563 | 52,1 | 0 |
| | | 625 | 2325 | | |
| | | 650 | 2500 | | |
| | | 300 | 1838 | | |
| | | Mittel 518 | Mittel 2556 | | |
| Verbindung der Formel I | 10.0 | 0 | 288 | 99,4 | 64,5 |
| | | 0 | 63 | | |
| | | 25 | 375 | | |
| | | 0 | 2100 | | |
| | | Mittel 6 | Mittel 706 | | |

Tabelle (Forsetzung)

| Wirkstoff | Dosis mg/kg | Anzahl Nematoden bei einzelnen Versuchstieren 14 - 16 Tage nach Behandlung | | Reduktion der Wurmzahl in % | |
|---|---|---|---|---|---|
| | | Haemonchus c. | Trichostrongylus c. | H.c. | T.c. |
| Fenbendazol/ Verbindung Formel I | 2.5<br>10.0 | 0<br>0<br>0<br>0<br>Mittel 0 | 63<br>88<br>438<br>- *<br>Mittel 196 | 100 | 90,1 |
| Fenbendazol/ Verbindung Formel I | 5.0<br>10.0 | 0<br>0<br>0<br>0<br>Mittel 0 | 0<br>188<br>88<br>0<br>Mittel 69 | 100 | 96,5 |
| Kontrolle | | 1575<br>750<br>500<br>1150<br>2000<br>525<br>Mittel 1083 | 2250<br>1788<br>1400<br>1975<br>2538<br>2000<br>Mittel 1991 | - | - |

* Testtier vorzeitig verunglückt

Bei den therapeutisch wirksamen Dosen der Wirkstoffkombinationen wurden an den Versuchstieren klinisch keine Toxizitätssymptome festgestellt.

Die erfindungsgemässen Wirkstoffkombinationen werden zur Bekämpfung parasitärer Helminthen bei Haus- und Nutztieren, wie Rindern, Schafen, Ziegen, Katzen und Hunden verwendet. Sie können den Tieren sowohl als Einzeldosis wie auch wiederholt verabreicht werden, wobei die einzelnen Gaben je nach Tierart vorzugsweise zwischen 0,5 und 100 mg pro kg Körpergewicht betragen. Durch eine protrahierte Verabreichung erzielt man in manchen Fällen eine bessere Wirkung oder man kann mit geringeren Gesamtdosen auskommen. Die Mittel können in Form von Lösungen, Emulsionen, Suspensionen (Drenchs), Pulver, Tabletten, Bolussen oder Kapseln peroral den Tieren verabreicht werden. Zur Bereitung dieser Applikationsformen dienen zum Beispiel übliche feste Trägerstoffe wie Kaolin, Talkum, Bentonit, Kochsalz, Calciumphosphat, Baumwollsaatmehl oder mit den Wirkstoffen nicht reagierende Flüssigkeiten wie Oele und andere, für den tierischen Organismus unschädliche Lösungs- und Verdünnungsmittel. Soweit die physikalischen und toxikologischen Eigenschaften von Lösungen oder Emulsionen dies zulassen, können die Wirkstoffkombinationen den Tieren auch beispielsweise subcutan injiziert oder mittels der Pouron-Methode appliziert werden. Ferner ist eine Verabreichung der Wirkstoffe an die Tiere auch mittels Lecksteinen (Salz) oder Molasse-Blöcken möglich.

Die Wirkstoffe bzw. sie enthaltenden Gemische können auch dem Futter oder den Tränken zugesetzt werden. Das Fertigfutter enthält die Wirkstoffkombinationen vorzugsweise in einer Konzentration von 0,005 bis 0,1 Gew.%.

Liegen die anthelmintischen Mittel in Form von Futterkonzentrat vor, so dienen als Hauptträgerstoffe Futtermaterialien wie beispielsweise Heu, Leistungsfutter, Futtergetreide oder Proteinkonzentrate. Dem Futter können ausser den Wirkstoffen noch Zusatzstoffe, Vitamine, Antibiotika, Chemotherapeutika oder andere Pestizide, vornehmlich Bakteriostatika, Fungistatika, Coccidiostatika oder auch Hormonpräparate, Stoffe mit anaboler Wirkung oder andere das Wachstum begünstigende, die Fleischqualität von Schlachttieren beeinflussende oder in anderer Weise für den Organismus nützliche Stoffe enthalten. Auch können sie mit Insektiziden und Akariziden kombiniert werden, wodurch ihre Wirkung verbreitert und an gegebene Umstände angepasst wird.

Die Herstellung der erfindungsgemässen anthelmintischen Mittel erfolgt in an sich bekannter Weise durch inniges Vermischen und Vermahlen der Wirkstoffkombinationen bestehend aus der Verbindung der Formel I und Verbindungen der Formel II mit geeigneten Trägerstoffen gegebenenfalls unter Zusatz von gegenüber den Wirkstoffen inerten Dispersions- oder Lösungsmitteln.

Die erfindungsgemässen anthelmintischen Mittel können beispielsweise in folgenden Zubereitungsformen angewendet werden :

Feste Zubereitungsformen : Granulate, Umhüllungsgranulate, Imprägnierungsgranulate und Homogengranulate sowie in Wasser dispergierbare Wirkstoffkonzentrate (Wettable Powder).

Flüssige Zubereitungsformen : Lösungen, Pasten, Emulsionen (insbesondere gebrauchsfertige Suspensionen (Drenches).

Die Korngrösse der Trägerstoffe beträgt für Stäubemittel und Spritzpulver zweckmässig bis ca. 0,1 mm und für Granulate 0,01-0,5 mm.

Die Konzentration der Wirkstoffkombinationen in den festen Zubereitungsformen beträgt 0,5 bis 80 %, in den flüssigen Aufarbeitungsformen 0,5 bis 50 %.

Diesen Gemischen können ferner die Wirkstoffkombinationen stabilisierende Zusätze und/oder nichtionische, anionaktive und kationaktive Stoffe zugegeben werden, die beispielsweise eine bessere Benetzbarkeit (Netzmittel) sowie Dispergierbarkeit (Dispergatoren) gewährleisten.

Beispiel : In Wasser dispergierbare Pulvermischung

25 Gew.-Teile Wirkstoffkombination von Verbindung der Formel I mit Verbindungen der Formel II werden in einem Mischapparat mit

7,5 Gew.-Teilen eines aufsaugenden Trägermaterials beispielweise Kielselsäure und
59,4 Gew.-Teilen eines Trägermaterials beispielsweise Bolus alba oder Kaolin und
0,5 Gew.-Teilen Oelsäure und
5,3 Gew.-Teilen Octylphenylpolyglykoläther
2,3 Gew.-Teilen Stearyl-benzimidazol-Derivate intensiv vermischt.

Diese Mischung wird auf einer Stift- oder Luftstrahlmühle bis zu einer Partikelgrösse von 5-15 μm gemahlen. Das so erhaltene Spritzpulver gibt in Wasser eine gute Suspension.

**Ansprüche**

1. Mittel zur Bekämpfung parasitärer Helminthen, dadurch gekennzeichnet, dass es als aktive Komponente eine Wirkstoffkombination bestehend aus mindestens einer Verbindung der Formel I

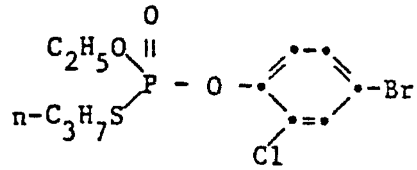

(I)

und mindestens einer Verbindung der Formel II

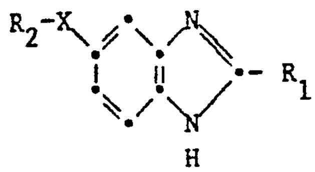

(II)

in welcher $R_1$ die Gruppe —NH—COO—$R_3$, worin $R_3$ einen Alkylrest mit 1 bis 4 Kohlenstoffatomen darstellt, $R_2$ einen Alkylrest mit 1 bis 4 Kohlenstoffatomen oder gegebenenfalls durch Halogen oder Alkyl substituiertes Phenyl und X : O oder S oder —S(O)— bedeuten, einschliesslich der Säureadditionssalze der unter die Formel II fallenden Verbindungen im Gewichtsverhältnis von 1 : 10 bis 10 : 1 zusammen mit geeigneten Trägerstoffen und/oder Verteilungs- und Verdünnungsmitteln enthält.

2. Mittel zur Bekämpfung parasitärer Helminthen gemäss Anspruch 1, dadurch gekennzeichnet, dass es als aktive Komponente eine Wirkstoffkombination bestehend aus einer Verbindung der Formel I

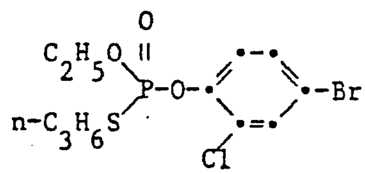

(I)

und einer Verbindung der Formel Ia

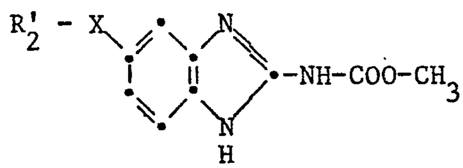

(Ia)

in welcher $R'_2$ einen n-Propyl-Rest oder einen Phenylrest darstellt und X: O oder S oder —S(O)— bedeutet, zusammen mit geeigneten Trägerstoffen und/oder Verteilungs- und Verdünnungsmitteln enthält.

3. Mittel zur Bekämpfung parasitärer Helminthen gemäss Anspruch 1, dadurch gekennzeichnet, dass es als aktive Komponente eine Wirkstoffkombination bestehend aus O-Aethyl-S-n-propyl-O-(2-chlor-4-bromphenyl)-phosphat und [5-(n-Propylthio)-1H-benzimidazol-2-yl]-carbaminsäuremethylester oder dessen Säureadditionssalz zusammen mit geeigneten Trägerstoffen und/oder Verteilungs- und Verdünnungsmitteln enthält.

4. Mittel zur Bekämpfung parasitärer Helminthen gemäss Anspruch 1, dadurch gekennzeichnet, dass es als aktive Komponente eine Wirkstoffkombination bestehend aus O-Aethyl-S-n-propyl-O-(2-chlor-4-bromphenyl)-phosphat und [5-(Phenylthio)-1H-benzimidazol-2-yl]-carbaminsäuremethylester oder dessen Säureadditionssalz zusammen mit geeigneten Trägerstoffen und/oder Verteilungs- und Verdünnungsmitteln enthält.

5. Mittel zur Bekämpfung parasitärer Helminthen gemäss Anspruch 1, dadurch gekennzeichnet, dass es als aktive Komponente eine Wirkstoffkombination bestehend aus O-Aethyl-S-n-propyl-O-(2-chlor-4-bromphenyl)-phosphat und [5-n-Propoxy-1H-benzimidazol-2-yl]-carbaminsäuremethylester oder dessen Säureadditionssalz zusammen mit geeigneten Trägerstoffen und/oder Verteilungs- und Verdünnungsmitteln enthält.

6. Mittel zur Bekämpfung parasitärer Helminthen gemäss Anspruch 1, dadurch gekennzeichnet, dass es als aktive Komponente eine Wirkstoffkombination bestehend aus O-Aethyl-S-n-propyl-O-(2-chlor-4-bromphenyl)-phosphat und [5-(Phenylsulfinyl)-1H-benzimidazol-2-yl]-carbaminsäuremethylester oder dessen Säureadditionssalz zusammen mit geeigneten Trägerstoffen und/oder Verteilungs- und Verdünnungsmitteln enthält.

7. Anthelmintische Mittel nach den Ansprüchen 1 bis 6, dadurch gekennzeichnet, dass in der

Wirkstoffkombination das Gewichtsverhältnis der Verbindung der Formel I zu Verbindungen der Formel II oder deren Säureadditionssalze 1 : 5 bis 5 : 1 beträgt.

8. Mittel gemäss den Ansprüchen 1 bis 7 zur Bekämpfung von resistenten Helminthen.

9. Mittel gemäss den Ansprüchen 1 bis 7 zur Bekämpfung von Nematoden.

10. Mittel gemäss den Ansprüchen 1 bis 7 zur Bekämpfung von resistenten Nematoden.

11. Mittel gemäss den Ansprüchen 1 bis 7 zur Bekämpfung von resistenten Haemonchus contortus und resistenten Trichostrongylus colubriformis.

12. Verfahren zur Herstellung eines anthelmintischen Mittels, dadurch gekennzeichnet, dass als aktive Komponente eine Wirkstoffkombination bestehend aus mindestens einer Verbindung der Formel I

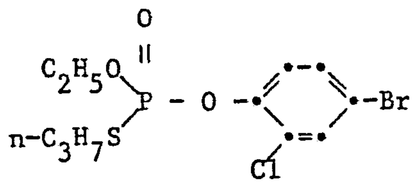

(I)

und mindestens einer Verbindung der Formel II

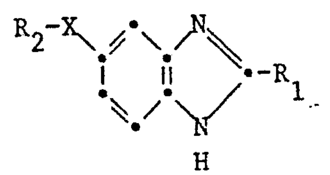

(II)

in welcher $R_1$ die Gruppe —NH—COO—$R_3$, worin $R_3$ einen Alkylrest mit 1 bis 4 Kohlenstoffatomen darstellt, $R_2$ einen Alkylrest mit 1 bis 4 Kohlenstoffatomen oder gegebenenfalls durch Halogen oder Alkyl substituiertes Phenyl und X : O oder S oder —S(O)— bedeuten, einschliesslich der Säureadditionssalze der unter die Formel II fallenden Verbindungen mit geeigneten festen oder flüssigen Trägerstoffen gegebenenfalls unter Zusatz von gegenüber den Wirkstoffen inerten Dispersions- oder Lösungsmitteln innig vermischt oder vermahlen wird.

13. Verfahren zur Herstellung eines anthelmintischen Mittels nach Anspruch 12, dadurch gekennzeichnet, dass die Wirkstoffkombination aus O-Aethyl-S-n-propyl-O-(2-chlor-4-bromphenyl)-phosphat und [5-(n-Propylthio)-1H-benzimidazol-2-yl]-carbaminsäuremethylester- oder dessen Säureadditionssalz besteht.

14. Verfahren zur Herstellung eines anthelmintischen Mittels nach Anspruch 12, dadurch gekennzeichnet, dass die Wirkstoffkombination aus O-Aethyl-S-n-propyl-O-(2-chlor-4-bromphenyl)-phosphat und [5-(Phenylthio)-1H-benzimidazol-2-yl]-carbaminsäuremethylester oder dessen Säureadditionssalz besteht.

15. Verfahren zur Herstellung eines anthelmintischen Mittels nach Anspruch 12, dadurch gekennzeichnet, dass die Wirkstoffkombination aus O-Aethyl-S-n-propyl-O-(2-chlor-4-bromphenyl)-phosphat und [5-n-Propoxy-1H-benzimidazol-2-yl]-carbaminsäuremethylester oder dessen Säureadditionssalz besteht.

16. Verfahren zur Herstellung eines anthelmintischen Mittels nach Anspruch 12, dadurch gekennzeichnet, dass die Wirkstoffkombination aus O-Aethyl-S-n-propyl-O-(2-chlor-4-bromphenyl)-phosphat und [5-(Phenylsulfinyl)-1H-benzimidazol-2-yl]-carbaminsäuremethylester oder dessen Säureadditionssalz besteht.

**Claims**

1. A composition for controlling parasitic helminths, which composition contains an active ingredient combination comprising at least one compound of the formula I

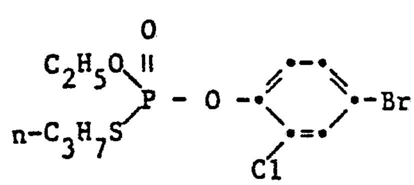

(I)

and at least one compound of the formula II

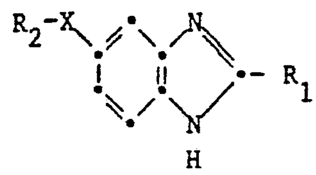

(II)

wherein $R_1$ is the —NH—COO—$R_3$ group, in which $R_3$ is an alkyl radical of 1 to 4 carbon atoms, $R_2$ is an alkyl radical of 1 to 4 carbon atoms, phenyl or phenyl substituted by halogen or alkyl, and X is —O—, —S— or —S(O)—, including an acid addition salt of a compound falling under the formula II, in the weight ratio of 1 : 10 to 10 : 1, together with suitable carriers and/or dispersants and diluents.

2. A composition for controlling parasitic helminths according to claim 1, which contains an active ingredient combination comprising a compound of the formula I

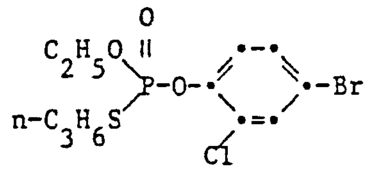

(I)

and a compound of the formula Ia

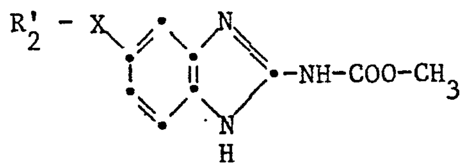

(Ia)

wherein $R'_2$ is a n-propyl radical or a phenyl radical, and X is —O—, —S— or —S(O)—, together with suitable carriers and/or dispersants and diluents.

3. A composition for controlling parasitic helminths according to claim 1, which contains an active ingredient combination comprising O-(4-bromo-2-chlorophenyl) O-ethyl S-propyl phosphorothioate and methyl-5-(n-propylthio)-1H-benzimidazol-2-ylcarbamate or an acid addition salt thereof, together with suitable carriers and/or dispersants and diluents.

4. A composition for controlling parasitic helminths according to claim 1, which contains an active ingredient combination comprising O-(4-bromo-2-chlorophenyl) O-ethyl S-propyl phosphorothioate and methyl 5-(phenylthio)-1H-benzimidazol-2-ylcarbamate or an acid addition salt thereof, together with suitable carriers and/or dispersants and diluents.

5. A composition for controlling parasitic helminths according to claim 1, which contains an active ingredient combination comprising O-(4-bromo-2-chlorophenyl) O-ethyl S-propyl phosphorothioate and methyl 5-(n-propoxy)-1H-benzimidazol-2-ylcarbamate or an acid addition salt thereof, together with suitable carriers and/or dispersants and diluents.

6. A composition for controlling parasitic helminths according to claim 1, which contains an active ingredient combination comprising O-(4-bromo-2-chlorophenyl) O-ethyl S-propyl phosphorothioate and methyl 5-(phenylsulfinyl)-1H-benzimidazol-2-ylcarbamate or an acid addition salt thereof, together with suitable carriers and/or dispersants and diluents.

7. An anthelmintic composition according to any one of claims 1 to 6, wherein the ratio of compound of formula I to compound of the formula II or acid addition salt thereof, in the active ingredient combination, is 1 : 5 to 5 : 1.

8. A composition according to any one of claims 1 to 7 for controlling resistant helminths.

9. A composition according to any one of claims 1 to 7 for controlling nematodes.

10. A composition according to any one of claims 1 to 7 for controlling resistant nematodes.

11. A composition according to any one of claims 1 to 7 for controlling resistant nematodes of the species Haemonchus contortus and Trichostrongylus colubriformis.

12. A process for the preparation of an anthelmintic composition, which comprises homogeneously mixing or grinding an active ingredient combination comprising at least one compound of the formula I

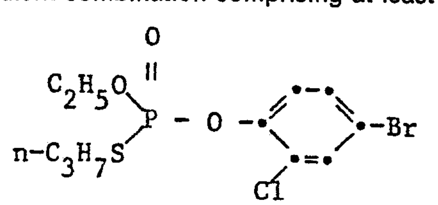

(I)

and at least one compound of the formula II

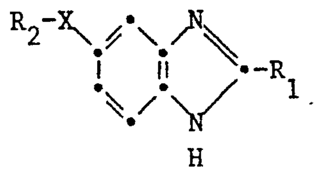

(II)

wherein $R_1$ is the —NH—COO—$R_3$ group, in which $R_3$ is an alkyl radical of 1 to 4 carbon atoms, $R_2$ is an alkyl radical of 1 to 4 carbon atoms, phenyl or phenyl substituted by halogen or alkyl, and X is —O—, —S— or —S(O)—, including an acid addition salt of a compound falling under the formula II, with suitable solid or liquid carriers, with or without the addition of dispersants or solvents which are inert to the active ingredients.

13. A process for the preparation of an anthelmintic composition according to claim 12, wherein the active ingredient combination comprises O-(4-bromo-2-chlorophenyl) O-ethyl S-propyl phosphorothioate and methyl 5-(n-propylthio)-1H-benzimidazol-2-ylcarbamate or an acid addition salt thereof.

14. A process for the preparation of an anthelmintic composition according to claim 12, wherein the active ingredient combination comprises O-(4-bromo-2-chlorophenyl) O-ethyl S-propyl phosphorothioate and methyl 5-(phenylthio)-1H-benzimidazol-2-ylcarbamate or an acid addition salt thereof.

15. A process for the preparation of an anthelmintic composition according to claim 12, wherein the active ingredient combination comprises O-(4-bromo-2-chlorophenyl) O-ethyl S-propyl phosphorothioate and methyl 5-(n-propoxy)-1H-benzimidazol-2-ylcarbamate or an acid addition salt thereof.

16. A process for the preparation of an anthelmintic composition according to claim 12, wherein the active ingredient combination comprises O-(4-bromo-2-chlorophenyl) O-ethyl S-propyl phosphorothioate and methyl 5-(phenylsulfinyl)-1H-benzimidazol-2-ylcarbamate or an acid addition salt thereof.

**Revendications**

1. Produit pour la lutte contre les helminthes parasitaires, caractérisé en ce qu'il contient en tant que composant actif une combinaison de substances actives consistant en au moins un composé de formule I

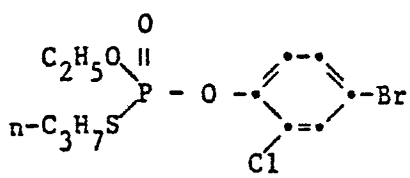

(I)

et au moins un composé de formule II

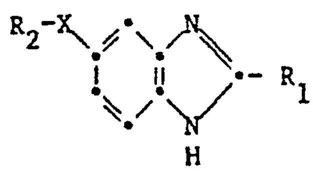

(II)

dans laquelle $R_1$ représente le groupe —NH—COO—$R_3$, $R_3$ représentant un groupe alkyle en $C_1$-$C_4$, $R_2$ représente un groupe alkyle en $C_1$-$C_4$ ou un groupe phényle éventuellement substitué par des halogènes ou des groupes alkyle et X représente O ou S ou —S(O)—, y compris les sels des composés de formule (II) formés par addition avec des acides, dans des proportions relatives en poids de 1 : 10 à 10 : 1, avec des véhicules et/ou dispersants et diluants appropriés.

2. Produit pour la lutte contre les helminthes parasitaires selon la revendication 1, caractérisé en ce qu'il contient en tant que composant actif une combinaison de substances actives consistant en un composé de formule I

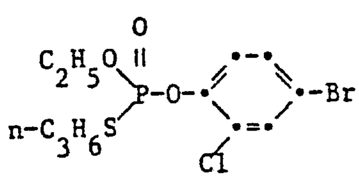

(I)

et un composé de formule Ia

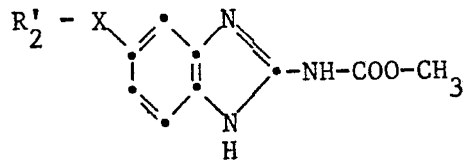

(Ia)

dans laquelle $R'_2$ représente un groupe n-propyle ou un groupe phényle et X représente O ou S ou —S(O)—, avec des véhicules et/ou dispersants et diluants appropriés.

3. Produit pour la lutte contre les helminthes parasitaires selon la revendication 1, caractérisé en ce qu'il contient en tant que composant actif une combinaison de substances actives consistant en phosphate d'O-éthyle, S-n-propyle et O-(2-chloro-4-bromophényle) et [5-(n-propylthio)-1H-benzimidazole-2-yl]-carbamate de méthyle ou un sel de ce composé formé par addition avec un acide, avec des véhicules et/ou dispersants et diluants appropriés.

4. Produit pour combattre les helminthes parasitaires selon la revendication 1, caractérisé en ce qu'il contient en tant que composant actif une combinaison de substances actives consistant en phosphate d'O-éthyle, S-n-propyle et O-(2-chloro-4-bromophényle) et [5-(phénylthio)-1H-benzimidazole-2-yl]-carbamate de méthyle ou un sel de ce composé formé par addition avec un acide, avec des véhicules et/ou dispersants et diluants appropriés.

5. Produit pour combattre les helminthes parasitaires selon la revendication 1, caractérisé en ce qu'il contient en tant que composant actif une combinaison de substances actives consistant en phosphate d'O-éthyle, S-n-propyle et O-(2-chloro-4-bromophényle) et [5-n-propoxy-1H-benzimidazole-2-yl]-carbamate de méthyle ou un sel de ce composé formé par addition avec un acide, avec des véhicules et/ou dispersants et diluants appropriés.

6. Produit pour combattre les helminthes parasitaires selon la revendication 1, caractérisé en ce qu'il contient en tant que composant actif une combinaison de substances actives consistant en phosphate d'O-éthyle, S-n-propyle et O-(2-chloro-4-bromophényle) et [5-(phénylsulfinyl)-1H-benzimidazole-2-yl]-carbamate de méthyle ou un sel de ce composé formé par addition avec un acide, avec des véhicules et/ou dispersants et diluants appropriés.

7. Produit anti-helminthique selon les revendications 1 à 6, caractérisé en ce que, dans la combinaison de substances actives, les proportions relatives en poids entre le composé de formule (I) et le composé de formule (II) ou son sel d'acide sont de 1 : 5 à 5 : 1.

8. Produit selon les revendications 1 à 7, pour la lutte contre les helminthes résistants.

9. Produit selon les revendications 1 à 7, pour la lutte contre les nématodes.

10. Produit selon les revendications 1 à 7, pour la lutte contre les nématodes résistants.

11. Produit selon les revendications 1 à 7, pour la lutte contre Haemonchus contortus résistant et Trichostrongylus colubriformis résistant.

12. Procédé de préparation d'un produit antihelminthique, caractérisé en ce que l'on mélange intimement ou on broie, en tant que composant actif, une combinaison de substances actives consistant en au moins un composé de formule I

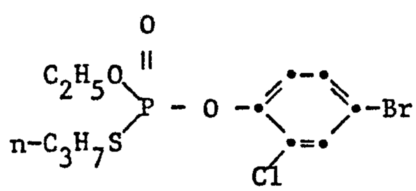

(I)

et au moins un composé de formule II

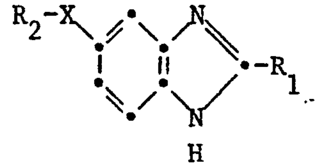

(II)

dans laquelle $R_1$ représente le groupe —NH—COO—$R_3$, $R_3$ représentant un groupe alkyle en $C_1$-$C_4$, $R_2$ représente un groupe alkyle en $C_1$-$C_4$ ou un groupe phényle éventuellement substitué par des halogènes ou des groupes alkyle, et X représente O ou S ou —S(O)—, y compris les sels des composés de formule (II) formés par addition avec des acides, avec des véhicules solides ou liquides appropriés, éventuellement avec adjonction d'agents dispersants ou de solvants inertes à l'égard des substances actives.

13. Procédé de préparation d'un produit antihelminthique selon la revendication 12, caractérisé en ce que la combinaison de substances actives consiste en phosphate d'O-éthyle, S-n-propyle et O-(2-chloro-4-bromophényle) et [5-(n-propylthio)-1H-benzimidazole-2-yl]-carbamate de méthyle ou un sel de ce composé formé par addition avec un acide.

14. Procédé de préparation d'un produit antihelminthique selon la revendication 12, caractérisé en ce que la combinaison de substances actives consiste en phosphate d'O-éthyle, S-n-propyle et O-(2-chloro-4-bromophényle) et [5-(phénylthio)-1H-benzimidazole-2-yl]-carbamate de méthyle ou un sel de ce composé formé par addition avec un acide.

15. Procédé de préparation d'un produit antihelminthique selon la revendication 12, caractérisé en ce que la combinaison de substances actives consiste en phosphate d'O-éthyle, S-n-propyle et O-(2-chloro-4-bromophényle) et [5-n-propoxy-1H-benzimidazole-2-yl]-carbamate de méthyle ou un sel de ce composé formé par addition avec un acide.

16. Procédé de préparation d'un produit antihelminthique selon la revendication 12, caractérisé en ce que la combinaison de substances actives consiste en phosphate d'O-éthyle, S-n-propyle et O-(2-chloro-4-bromophényl) et [5-(phénylsulfinyl)-1H-benzimidazole-2-yl]-carbamate de méthyle ou un sel de composé formé par addition avec un acide.